(19) Europäisches Patentamt
European Patent Office
Office européen des brevets

(11) Veröffentlichungsnummer: **0 257 049**
**B1**

(12) # EUROPÄISCHE PATENTSCHRIFT

(45) Veröffentlichungstag der Patentschrift: **01.08.90**

(51) Int. Cl.$^5$: **C 07 C 255/00,**
C 07 C 255/45, C 09 K 19/30

(21) Anmeldenummer: **87901019.7**

(22) Anmeldetag: **31.01.87**

(86) Internationale Anmeldenummer:
**PCT/DE87/00037**

(87) Internationale Veröffentlichungsnummer:
**WO 87/05015 27.08.87 Gazette 87/19**

(54) **CHIRALE VERBINDUNGEN.**

(30) Priorität: **17.02.86 DE 3604905**

(43) Veröffentlichungstag der Anmeldung:
**02.03.88 Patentblatt 88/09**

(45) Bekanntmachung des Hinweises auf die Patenterteilung:
**01.08.90 Patentblatt 90/31**

(84) Benannte Vertragsstaaten:
**CH DE FR GB LI**

(56) Entgegenhaltungen:
**EP-A-0 063 003**
**WO-A-86/05486**
**DE-A-3 221 941**
**JP-A-6 130 564**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **EIDENSCHINK, Rudolf**
**Konrad Adenauer Strasse 1**
**D-6109 Mühltal 1 (DE)**
Erfinder: **HOPF, Reinhard**
**Wolfsgasse 3**
**D-6432 Heringen (DE)**
Erfinder: **POETSCH, Eike**
**Am Buchwald 4**
**D-6109 Mühltal 6 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 257 049 B1

**Beschreibung**

Die Erfindung betrifft optisch aktive Verbindungen der Formel I

$$R^1—A^1—Z^1—A^2—R^2 \qquad I$$

worin

$$A^1 \; A, \; A^4—A \text{ oder } A—A^4,$$

$R^1$ und $R^2$ jeweils H, eine Alkylgruppe mit 1—12 C-Atomen, worin auch eine oder zwei nicht benachbarte $CH_2$-Gruppen durch O-Atome und/oder —CO-Gruppen und/oder —CO—O-Gruppen und/oder —CH=CH-Gruppen und/oder —CHHalogen- und/oder —CHCN-Gruppen ersetzt sein können, F, Cl, Br, CN oder $R^3—A^3—Z^2$,

A eine in 2-, 3-, 5- oder 6-Stellung durch Hydroxy, Halogen, Nitril und/oder eine Alkylgruppe oder eine fluorierte Alkylgruppe mit jeweils 1—10 C-Atomen, worin auch eine oder zwei nicht benachbarte $CH_2$-Gruppen durch —O—, —CO—, —O—CO—, —CO—O— und/oder —CH=CH— ersetzt sein können, substituierte 1,4-Cyclohexylen- oder 1,4-Cyclohexenylen-Gruppe, worin auch eine oder zwei nicht benachbarte $CH_2$-Gruppen durch —O— und/oder —S— ersetzt sein können, die gegebenenfalls auch in 1- und/oder 4-Stellung durch Hydroxy, Halogen, Nitril und/oder eine Alkylgruppe oder eine fluorierte Alkylgruppe mit jeweils 1—4 C-Atomen, worin auch eine oder zwei nicht benachbarte $CH_2$-Gruppen durch —O—, —CO—, —O—CO—, —CO—O— und/oder —CH=CH— ersetzt sein können, substituiert sein kann,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte $CH_2$-Gruppen durch O-Atome und/oder S-Atome ersetzt sein können, Piperidin-1,4-diyl, 1,4-Bicyclo(2,2,2)-octylen-, Decahydronaphthalin-2,6-diyl- oder 1,2,3,4-Tetrahydronaphthalin-2,6-diyl-Gruppen,

$Z^1$ und $Z^2$ jeweils —CO—O—, —O—CO—, —$CH_2CH_2$—, $OCH_2$—, —$CH_2O$— oder eine Einfachbindung,

$R^3$ H, eine Alkylgruppe mit 1—12, worin auch eine oder zwei nicht benachbarte $CH_2$-Gruppen durch O-Atome und/oder —CO-Gruppen und/oder —CO—O-Gruppen und/oder —CH=CH-Gruppen ersetzt sein können, F, Cl, Br oder CN

bedeutet.

Die Verbindungen der Formel I können wie ähnliche in DE—OS—35 15 373 beschriebene Verbindungen als Komponenten chiraler getilteter smektischer flüssigkristalliner Phasen verwendet werden.

In der EP—A—063003 und der nichtveröffentlichten WO—A—86 05 485 werden einige Verbindungen der Formel I jedoch in der vacemischen Form als Komponenten nematischer flüssigkristalliner Phasen beschrieben. In der JP—A—61 30564 und der DE—A—32 21 941 werden ähnliche Verbindungen beschrieben, welche jedoch keiner chiralität aufweisen und somit nicht als Komponenten chiraler getilteten Phasen verwendet werden Können.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L. A. Beresnev et al., Mol. Cryst. Liq. Cryst. *89,* 327 (1982); H. R. Brand et al., J. Physique *44,* (lett.), L—771 (1983). Solche Phasen können als Dielektrika für schnell schaltende Displays verwendet werden, die auf dem von Clark und Lagerwall beschriebenen Prinzip der SSFLC-Technologie (N. A. Clark und S. T. Lagerwall, Appl. Phys. Lett. *36,* 899 (1980); USP 4,367,924) auf der Basis der ferroelektrischen Eigenschaften der chiralen getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1—2 μm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN—LCD's), die auf nematischen Flüssigkristallen basieren.

Ein großer Nachteil für viele Anwendungen der derzeit verfügbaren Materialien mit chiralen getilteten smektischen Phasen (wie z.B. Sc*) ist, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J. M. Geary, SID-Tagung, Orlando/Florida, April/Mai 1985, Vortrag 8.3).

Es wurde nun gefunden, daß die Verwendung von Verbindungen der Formel I als Komponenten chiraler getilteter smektischer Mischungen die erwähnten Nachteile wesentlich vermindern kann. Die Verbindungen der Formel I sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc*-Phasenbereichen, negativer oder auch positiver dielektrischer Anisotropie, niedriger optischer Anisotropie, günstiger Pitchhöhe und für derartige Phasen hohen Werten für die spontane Polarisation herstellbar. P ist die spontane Polarisation in $nC/cm^2$.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basis-materialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und weisen niedrige Werte der optischen Anisotropie auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel I als Komponenten chiraler getilteter smektischer Phasen vorteilhaft eingesetzt werden. Chemisch, thermisch und gegen Licht sind sie sehr stabil.

Gegenstand der Erfindung sind somit die optisch aktiven Verbindungen der Formel I sowie die Verwendung der Verbindungen der Formel I als Komponenten flüssigkristalliner Phasen.

Gegenstand der Erfindung sind auch chirale getiltete smektiche flüssigkristalline Phasen mit einem Gehalt an mindestens einer optisch aktiven Verbindung der Formel I mit mindestens einem asymmetrischen Kohlenstoffatom.

Gegenstand der Erfindung sind ferner solche Phasen mit einem Gehalt an mindestens einer Verbindung der Formel I sowie Flüssigkristallanzeigeelemente, insbesondere elektrooptische Anzeigeelemente, die derartige Phasen enthalten.

Der Einfachheit halber bedeuten im folgenden Ph eine 1,4-Phenylengruppe, worin auch eine oder zwei CH-Gruppen durch N ersetzt sein können, Cy eine 1,4-Cyclohexylengruppe, worin auch eine oder zwei nicht benachbarte $CH_2$-Gruppen durch O-Atome ersetzt sein können und Bi eine Bicyclo(2,2,2)octylengruppe.

Vor- und nachstehend haben $R^1$, $R^2$, $R^3$, $A^1$, $A^2$, $A^3$, $A^4$, A, $Z^1$ und $Z^2$ die angegebene Bedeutung, sofern nicht ausdrücklich etwas anderes vermerkt ist.

Die Verbindungen der Formel I umfassen dementsprechend insbesondere Verbindungen der Teilformeln Ia und Ib (mit zwei Ringen)

$R^1$—A—$A^2$—$R^2$ Ia
$R^1$—A—$Z^1$—$A^2$—$R^2$ Ib

Ie bis Ik (mit drei Ringen),

$R^1$—$A^4$—A—$A^2R^2$ Ie
$R^1$—A—$A^4$—$A^2$—$R^2$ If
$R^1$—$A^4$—A—$Z^1$—$A^2$—$R^2$ Ig
$R^1$—A—$A^4$—$Z^1$—$A^2$—$R^2$ Ih
$R^1$—A—$Z^1$—$A^2$—$A^3$—$R^3$ Ii
$R^3$—$A^3$—$Z^2$—A—$Z^1$—$A^2$—$R^2$ Ij
$R^1$—A—$Z^1$—$A^2$—$Z^2$—$A^3$—$R^3$ Ik

Ip bis Iaa (mit vier Ringen),

$R^1$—$A^4$—A—$A^2$—$A^3$—$R^3$ Ip
$R^1$—A—$A^4$—$A^2$—$A^3$—$R^3$ Iq
$R^3$—$A^3$—$Z^2$—$A^4$—A—$A^2$—$R^2$ Ir
$R^3$—$A^3$—$A^4$—A—$Z^1$—$A^2$—$R^2$ Is
$R^1$—A—$A^4$—$A^2$—$Z^2$—$A^3$—$R^3$ It
$R^1$—A—$A^4$—$Z^1$—$A^2$—$A^3$—$R^3$ Iu
$R^1$—$A^4$—A—$Z^1$—$A^2$—$A^3$—$R^3$ Iv
$R^1$—$A^4$—A—$Z^1$—$A^2$—$Z^2$—$A^3$—$R^3$ Iw
$R^1$—A—$A^4$—$Z^1$—$A^2$—$Z^2$—$A^3$—$R^3$ Ix
$R^3$—$A^3$—$Z^2$—$A^4$—A—$Z^1$—$A^2$—$R^2$ Iy
$R^3$—$A^3$—$Z^2$—A—$A^4$—$Z^1$—$A^2$—$R^2$ Iz
$R^3$—$A^3$—$Z^2$—A—$Z^1$—$A^2$—$Z^2$—$A^3$—$R^3$ Iaa

sowie Iab bis Iak (mit fünf Ringen),

| | |
|---|---|
| $R^3—A^3—Z^2—A^4—A—Z^1—A^2—Z^2—A^3—R^3$ | Iab |
| $R^3—A^3—Z^2—A—A^4—Z^1—A^2—Z^2—A^3—R^3$ | Iac |
| $R^3—A^3—A^4—A—Z^1—A^2—Z^2—A^3—R^3$ | Iad |
| $R^3—A^3—Z^2—A^4—A—A^2—Z^2—A^3—R^3$ | Iae |
| $R^3—A^3—Z^2—A^4—A—Z^1—A^2—A^3—R^3$ | Iaf |
| $R^3—A^3—A—A^4—A^2—Z^2—A^3—R^3$ | Iag |
| $R^3—A^3—Z^2—A—A^4—A^2—A^3—R^3$ | Iah |
| $R^3—A^3—A^4—A—Z^1—A^2—A^3—R^3$ | Iai |
| $R^3—A^3—A^4—A—A^2—A^3—R^3$ | Iaj |
| $R^3—A^3—A—A^4—A^2—A^3—R^3$ | Iak |

Darunter sind diejenigen der Formeln Ia, Ib, Ie, If, Ig, Ih, Ii, Ip und Iq besonders bevorzugt.

Die bevorzugten Verbindungen der Formel Ia umfassen solche der Teilformeln Ia1 bis Ia3:

| | |
|---|---|
| $R^1—A—Ph—R^2$ | Ia1 |
| $R^1—A—Cy—R^2$ | Ia2 |
| $R^1—A—Bi—R^2$ | Ia3 |

Darunter sind diejenigen der Teilformeln Ia1 und Ia2 besonders bevorzugt.

Die bevorzugten Verbindungen der Formel Ib umfassen solche der Teilformeln Ib1 bis Ib3:

| | |
|---|---|
| $R^1—A—Z^1—Ph—R^2$ | Ib1 |
| $R^1—A—Z^1—Cy—R^2$ | Ib2 |
| $R^1—A—Z^1—Bi—R^2$ | Ib3 |

Darunter sind diejenigen der Teilformeln Ib1 und Ib2, insbesondere diejenigen worin $Z^1$ —CO—O—, O—CO— oder $—CH_2CH_2—$ bedeutet, besonders bevorzugt.

Die bevorzugten Verbindungen der Formel Ie umfassen solche der Teilformeln Ie1 und Ie2:

| | |
|---|---|
| $R^1—Cy—A—Cy—R^2$ | Ie1 |
| $R^1—Cy—A—Ph—R^2$ | Ie2 |

Die bevorzugten Verbindungen der Formel If umfassen solche der Teilformeln If1 bis If4:

| | |
|---|---|
| $R^1—A—Cy—Cy—R^2$ | If1 |
| $R^1—A—Ph—Ph—R^2$ | If2 |
| $R^1—A—Ph—Cy—R^2$ | If3 |
| $R^1—A—Cy—Ph—R^2$ | If4 |

Darunter sind diejenigen der Teilformeln If1 und If4 besonders bevorzugt.

Die bevorzugten Verbindungen der Formel Ig umfassen solche der Teilformeln Ig1 bis Ig3:

| | |
|---|---|
| $R^1—Cy—A—Z^1—Cy—R^2$ | Ig1 |
| $R^1—Cy—A—Z^1—Ph—R^2$ | Ig2 |
| $R^1—Ph—A—Z^1—Cy—R^2$ | Ig3 |

Darunter sind diejenigen der Teilformeln Ig1, insbesondere diejenigen worin $Z^1$ —CO—O—, —O—CO— oder $—CH_2CH_2—$ bedeutet, besonders bevorzugt.

Die bevorzugten Verbindungen der Formel Ih umfassen solche der Teilformeln Ih1 bis Ih4:

| | |
|---|---|
| $R^1—A—Cy—Z^1—Cy—R^2$ | Ih1 |
| $R^1—A—Ph—Z^1—Ph—R^2$ | Ih2 |
| $R^1—A—Ph—Z^1—Cy—R^2$ | Ih3 |
| $R^1—A—Cy—Z^1—Ph—R^2$ | Ih4 |

Darunter sind diejenigen der Teilformeln Ih1, Ih2 und Ih3, insbesondere diejenigen worin $Z^1$ —CO—O—, —O—CO— oder $—CH_2CH_2—$, insbesondere —CO—O—, bedeutet, besonders bevorzugt.

Die bevorzugten Verbindungen der formel Ii umfassen solche der Teilformeln Ii1 und Ii4:

| | |
|---|---|
| $R^1—A—Z^1—Cy—Cy—R^3$ | Ii1 |
| $R^1—A—Z^1—Ph—Cy—R^2$ | Ii2 |
| $R^1—A—Z^1—Cy—Ph—R^2$ | Ii3 |
| $R^1—A—Z^1—Ph—Ph—R^2$ | Ii4 |

Darunter sind diejenigen besonders bevorzugt, worin $Z^1$ —O—CO—, —CO—O— oder $—CH_2CH_2—$, insbesondere bevorzugt $—CH_2CH_2—$, bedeutet.

Die bevorzugten Verbindungen der Formel Ip umfassen solche der Teilformeln Ip1 und Ip2:

| | |
|---|---|
| $R^1—Cy—A—Ph—Ph—R^3$ | Ip1 |
| $R^1—Cy—A—Ph—Cy—R^3$ | Ip2 |

Die bevorzugten Verbindungen der Formel Iq umfassen solche der Teilformeln Iq1 und Iq2:

$R^1$—A—Ph—Ph—Cy—$R^3$ Iq1
$R^1$—A—Ph—Cy—Cy—$R^3$ Iq2

In den Verbindungen der vor- und nachstehenden Formeln bedeuten $R^1$, $R^2$ bzw. $R^3$ vorzugsweise Alkyl, ferner Alkoxy- oder eine andere Oxaalkylgruppe.

Weiterhin bevorzugt sind Verbindungen der vor- und nachstehenden Formeln worin einer der Reste $R^1$, $R^2$ bzw. $R^3$ —CO—Alkyl, —CO—O—Alkyl, —OCO—Alkyl oder —OCOO—Alkyl und der andere Alkyl oder Alkoxy bedeutet.

In den bevorzugten Verbindungen der vor- und nachstehenden Formeln können die Alkylreste, in denen auch eine $CH_2$-Gruppe (Alkoxy bzw. Oxaalkyl) durch ein O-Atom ersetzt sein kann, geradkettig oder verzweigt sein. Vorzugsweise sind sie geradkettig, haben 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

$A^2$, $A^3$ und $A^4$ sind bevorzugt Cy oder Ph. In den Verbindungen der vor- und nachstehenden Formeln bedeutet Ph vorzugsweise eine 1,4-Phenylen- oder eine Pyrimidin-2,5-diylgruppe, insbesondere bevorzugt 1,4-Phenylen. Cy bedeutet vorzugsweise eine 1,4-Cyclohexylengruppe.

$Z^1$ und $Z^2$ sind bevorzugt Einfachbindungen, in zweiter Linie bevorzugt —O—CO—, —CO—O— oder —$CH_2CH_2$-Gruppen.

A ist vorzugsweise eine in 2-, 3-, 5- oder 6-Stellung durch Hydroxy, Halogen, Nitril und/oder Alkylgruppe mit vorzugsweise 1—5 C-Atomen, worin auch eine oder zwei nicht benachbarte $CH_2$-Gruppen vorzugsweise durch —O— und/oder —CO— ersetzt sein können, substituierte 1,4-Cyclohexylengruppe, die gegebenenfalls auch in 1- und/oder 4-Stellung durch Halogen, Nitril und/oder eine Alkylgruppe mit 1—4 C-Atomen, insbesondere durch Nitril, substituiert sein kann.

A ist bevorzugt eine Gruppe ausgewählt aus den Formeln (A) bis (C),

(A)

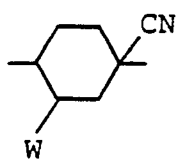

(B)

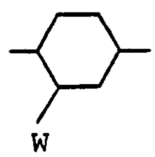

(C)

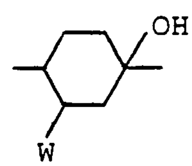

worin W OH, Halogen, Nitril, Alkyl, Alkoxy, Oxaalkyl, Alkanoyl, Alkanoyloxy oder Alkoxycarbonyl mit jeweils 1 bis 10 C-Atomen bedeutet.

Bevorzugt sind ferner Verbindungen der Formeln (A)—(C), worin W vorzugsweise Alkyl, Alkoxy, Halogen oder Nitril bedeutet. W ist insbesondere bevorzugt $CH_3$.

A umfaßt auch die Spiegelbilder der Formeln (A) bis (C).

$R^1$—A ist weiterhin bevorzugt eine Gruppe der Formeln (D)—(F),

(D)

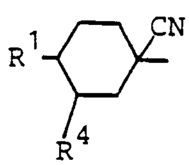

(E)

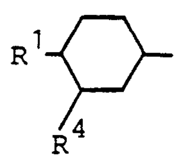

(F)

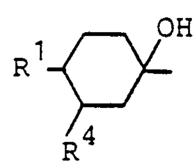

worin $R^4$ vorzugsweise n-Alkyl, n-Alkoxy, n-Alkoxycarbonyl, (jeweils vorzugsweise mit 1 bis 10, insbesondere mit 1 bis 7, C-Atomen) oder CN bedeutet. $R^1$ bedeutet hier H.

Besonders bevorzugt sind (D) und (F), worin $R^4$ vorzugsweise $CH_3$ bedeutet.

Bevorzugte Bedeutung von W ist F, Cl, CN, —$CH_3$ und —$CH_2CH_3$. Besonders bevorzugt sind die Gruppen (A) und (B).

Besonders bevorzugt für $Z^1$ ist —CO—O—, —O—CO— oder —$CH_2CH_2$—, insbesondere die —$CH_2CH_2$-Gruppe.

Verbindungen der vor- und nachstehenden Formeln mit verzweigten Flügelgruppen $R^1$, $R^2$ bzw. $R^3$ können gelegentlich wegen einer besseren Löslichkeit in den üblichen flüssigkristallinen Basismaterialien von Bedeutung sein. Verzweigte Gruppen dieser Art enthalten in der Regel nicht mehr als eine Kettenverzweigung.

Bevorzugte verzweigte Reste sind Isopropyl, 2-Butyl (= 1-Methylpropyl), Iosbutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl,

Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formel I sowie Ia bis Iq sind diejenigen bevorzugt, in denen mindestens einer der darin enthaltenen Reste eine der angegebenen bevorzugten Bedeutungen hat.

In den Verbindungen der vorstehenden genannten Formeln sind diejenigen Stereoisomeren bevorzugt, in denen die Substituenten $R^1$—, $R^1$—$A^4$—, $R^2$—$A^2$—$Z^1$— bzw. $R^2$—$A^2$—$Z^1$—$A^4$— in 1- und 4-Position des Ringes A trans-ständig sind und die äquatoriale Stellung einnehmen, während der gegebenenfalls vorhandene zusätzliche Substituent an A in 1- oder 4-Position eine axiale Stellung einimmt. Diese sind in der Regel stabiler; in vielen Fällen lassen sich die cis-Verbindungen (oder Gemische) durch Behandeln mit einer Base z.B. mit K-tert.-Butylat in einem inerten Lösungsmittel wie Dimethylsulfoxid, in die trans-Verbindungen umwandeln.

Der Substituent W in den Gruppen der Formeln (A) bis (C) kann äquatoriale oder axiale Stellungen einnehmen.

Diejenigen der vorstehend genannten Formeln, die eine oder mehrere Gruppen DiO, Dit, Pip und/oder Pyr enthalten, umschließen jeweils die beiden möglichen 2,5-(Dio, Dit, Pyr) bzw. 1,4-Stellungsisomeren (Pip).

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genanntgen Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C—C-Bindungen enthält, reduziert.

Als reduzierbare Gruppen kommen vorzugsweise —CH=CH-gruppen in Betracht, ferner z.B. freie oder veresterte Hydroxygruppen, aromatisch gebundene Halogenatome oder Carbonylgruppen. Bevorzugte Ausgangsstoffe für die Reduktion entsprechen der Formel I, können aber an Stelle einer —$CH_2$—CH-Gruppe eine —CH=CH-Gruppe und/oder an Stelle einer —$CH_2$-Gruppe eine —CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder —$CH_2CH_2$-Brücken enthalten, reduziert werden.

Weiterhin sind Reduktionen mit komplexen Hydriden möglich. Beispielsweise können Arylsulfonyloxygruppen mit $LaAlH_4$ reduktiv entfernt werden, insbesondere p-Toluol-sulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z.B. aus 1-Cyancyclohexenderivaten die entsprechenden Cyclohexanderivate.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1—4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedinungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogen-carbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa −25° und +20°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I können entsprechende Säureamide, z.B. solche in denen an Stelle des Restes X eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindungen mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol, oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßg zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Cyclohexanonderivate der Formel I sind weiterhin durch säurekatalysierte Umlagerungen der entsprechenden Epoxide nach literaturbekannten Verfahren, z.B. durch Behandeln mit $BF_3$-Etherat, zugänglich. Die Epoxide sind durch Epoxidierung der entsprechenden Cyclohexanderivate nach Standardverfahren erhältlich.

Zur Herstellung von Nitrilen der Formel I können auch entsprechende Chlor- oder Bromverbindungen der Formel I mit einem Cyanid umgesetzt werden, zweckmaßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Die optisch aktiven Verbindungen der Formel I erhält man durch den Einsatz entsprechender optisch

aktiver Ausgangsmaterialien und/oder durch Trennung der optischen Antipoden mittels Chromatographie nach bekannten Methoden.

Die erfindungsgemäßen Phasen enthalten vorzugsweise mindestens drei, insbesondere mindestens fünf Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der chiralen Basismischung können 1 bis 50%, vorzugsweise 10 bis 25%, der Basismischung ausmachen. Als weitere Komponenten mit betragsmäßig kleiner positiver oder negativer dielektrischer Anisotropie eignen sich Verbindungen der Teilformeln Va bis Vp:

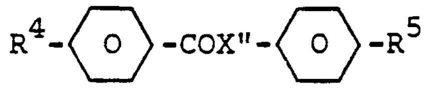 Va

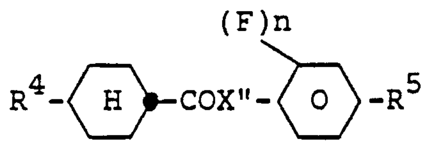 Vb

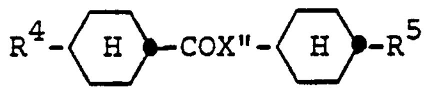 Vc

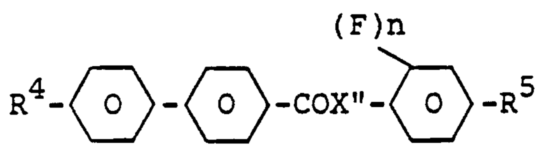 Vd

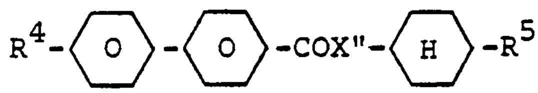 Ve

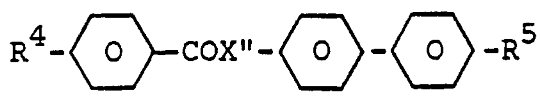 Vf

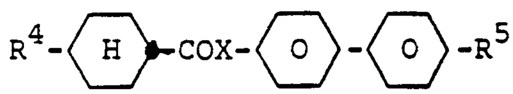 Vg

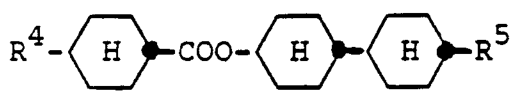 Vh

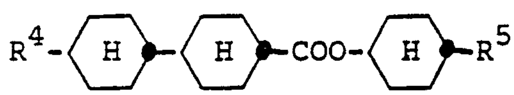 Vi

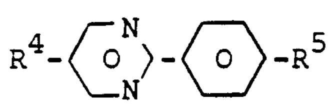 Vj

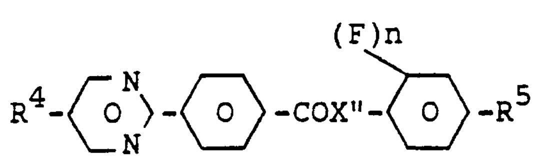 Vk

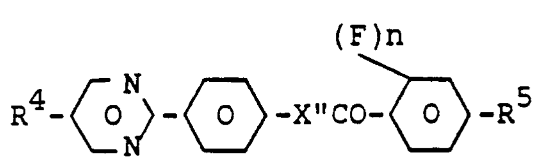 Vl

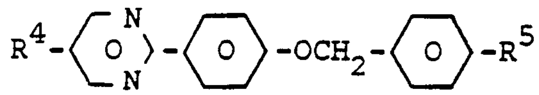

Vm

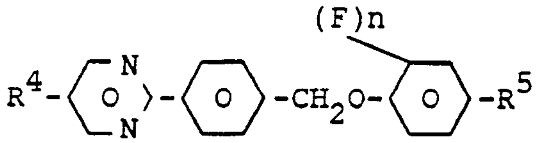

Vn

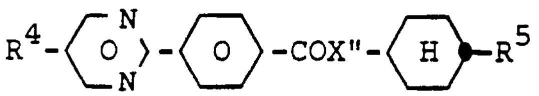

Vo

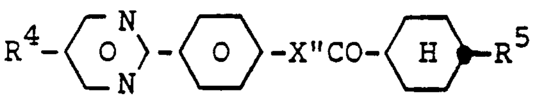

Vp

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X'' ist O oder S, vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5%, vorzugsweise 1 bis 3%, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

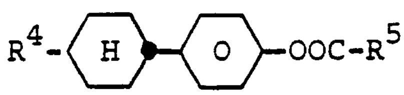

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement M, N oder O.

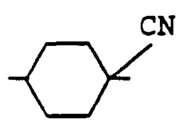

M

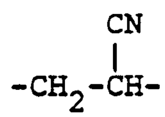

N

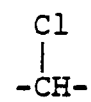

O

Bevorzugte Verbindungen dieser Art entsprechen den Formeln VIb und VIc:

R'—Q¹—CH₂—CH—Q²—R''
|
CN

VIb

R'—Q³—Q⁴—R'''

VIc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

Cl
|
—CH*— oder

CN
|
—CH*—.

Besonders bevorzugte Verbindungen der Formel VIc sind diejenigen der Formel VIc':

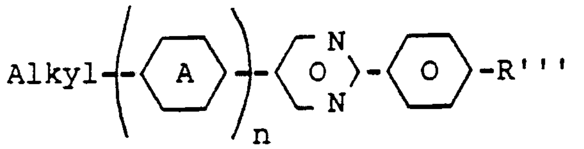

VIc'

worin A 1,4-Phenylen oder trans-1,4-Cyclohexylen und n 0 oder 1 bedeutet.

Die Verbindungen der Formel I eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nametischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'—L—G—E—R'' I'

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO— |

oder eine C—C-Einfachbindung,

Y Halogen, vorzugsweise Chlor, oder —CN, und R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95%, einer oder mehrerer Verbindungen der Formel I. Weiterhin bevorzugt sind erfindungsgemäße flüssigkristalline Phasen, enthaltend 0,1—40, vorzugsweise 0,5—30% einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst Band *24,* Seiten 249—258 (1975)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE—OS—22 09 127, 22 40 864, 23 21 632, 23 38 281, 24 50 088, 26 37 430, 28 53 728 and 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

a) Zu einer Lösung von 36 g (+)-3-Methylcyclohexanon in 630 ml THF gibt man 105 g 2,4,6-Triisopropylbenzolsulfonsäurehydrazid und rührt 2 Stunden bei Raumtemperatur. Hierauf werden 450 ml Methanol und 65 g KCN zugefügt und 2 Stunden m Rückfluß erhitzt. Nach üblicher Aufarbeitung und anschließender fraktionierter Destillation erhält man ein Gemisch der diasteromeren 3-Methylcyclohexancarbonitrile.

b) Zu einer Lösung von 17 g 3-Methylcyclohexancarbonitril in 140 ml THF gibt man bei −78° eine Lithiumdiisopropylamid-Lösung (hergestellt aus 140 ml THF, 16 g Diisopropylamin und 100 ml 1,6 molarer Butyllithium-Lösung in Hexan), nach 2 Stunden 25 g Diethylcarbonat und rührt noch eine Stunde. Nach Erwärmen auf Raumtemperatur und Gießen auf 300 ml Eiswasser, wird mit verd. Salzsäure neutralisiert. Nach üblicher Aufarbeitung und Destillation erhält man ein Gemisch der diastereomeren 3-Methyl-1-cyan-cyclohexancarbonsäureethylester.

Dieser Ester wird durch Erwärmen mit ethanolischer Kalilauge verseift. Nach Neutralisation, Extraktion mit Methyl-tert.-butylether und Abdestillieren des Lösungsmittels erhält man das Gemisch der diastereomeren 1-Cyan-3-methylcyclohexancarbonsäuren.

c) Zu einem Gemisch aus 16 g 1-Cyan-3-methyl-cyclohexancarbonsäure, 25 g 4'-Hexyl-biphenylol-4, 1,2 g 4-Diethylaminopyridin und 200 ml Methylenchlorid gibt man bei Raumtemperatur eine Lösung von 23 g Dicyclohexylcarbodiimid in 50 ml $CH_2Cl_2$ und rührt 2 Stunden. Nach üblicher Aufarbeitung und säulenchromatographischer Trennung (Kieselgel/Toluol) erhält man als Reinprodukt durch Umkristallisation das 4'-Hexylbiphenyl-4-yl-1-cyan-3-methylcyclohexancarboxylat, Fp. 51°, $[\alpha]_D^{20} = +2{,}2°$.

Analog weren hergestellt:

4'-Ethylbiphenyl-4-yl-1-cyan-3-methylcyclohexancarboxylat
4'-Propylbiphenyl-4-yl-1-cyan-3-methylcyclohexancarboxylat
4'-Butylbiphenyl-4-yl-1-cyan-3-methylcyclohexancarboxylat
4'-Pentylbiphenyl-4-yl-1-cyan-3-methylcyclohexancarboxylat
4'-Heptylbiphenyl-4-yl-1-cyan-3-methylcyclohexancarboxylat
4'-Ethoxybiphenyl-4-yl-1-cyan-3-methylcyclohexancarboxylat
4'-Propoxybiphenyl-4-yl-1-cyan-3-methylcyclohexancarboxylat
4'-Butoxybiphenyl-4-yl-1-cyan-3-methylcyclohexancarboxylat
4'-Pentoxybiphenyl-4-yl-1-cyan-3-methylcyclohexancarboxylat
4'-Hexoxybiphenyl-4-yl-1-cyan-3-methylcyclohexancarboxylat
4'-Heptoxybiphenyl-4-yl-1-cyan-3-methylcyclohexancarboxylat
4'-Ethylbiphenyl-4-yl-1-cyan-3-ethylcyclohexancarboxylat
4'-Propylbiphenyl-4-yl-1-cyan-3-ethylcyclohexancarboxylat
4'-Butylbiphenyl-4-yl-1-cyan-3-ethylcyclohexancarboxylat
4'-Pentylbiphenyl-4-yl-1-cyan-3-ethylcyclohexancarboxylat
4'-Hexylbiphenyl-4-yl-1-cyan-3-ethylcyclohexancarboxylat
4'-Heptylbiphenyl-4-yl-1-cyan-3-ethylcyclohexancarboxylat
4'-Ethoxybiphenyl-4-yl-1-cyan-3-ethylcyclohexancarboxylat
4'-Propoxybiphenyl-4-yl-1-cyan-3-ethylcyclohexancarboxylat
4'-Butoxybiphenyl-4-yl-1-cyan-3-ethylcyclohexancarboxylat
4'-Pentoxybiphenyl-4-yl-1-cyan-3-ethylcyclohexancarboxylat
4'-Hexoxybiphenyl-4-yl-1-cyan-3-ethylcyclohexancarboxylat
4'-Heptoxybiphenyl-4-yl-1-cyan-3-ethylcyclohexancarboxylat
4'-Ethylbiphenyl-4-yl-1-cyan-3-chlorcyclohexancarboxylat
4'-Propylbiphenyl-4-yl-1-cyan-3-chlorcyclohexancarboxylat
4'-Butylbiphenyl-4-yl-1-cyan-3-chlorcyclohexancarboxylat
4'-Pentylbiphenyl-4-yl-1-cyan-3-chlorcyclohexancarboxylat
4'-Hexylbiphenyl-4-yl-1-cyan-3-chlorcyclohexancarboxylat
4'-Heptylbiphenyl-4-yl-1-cyan-3-chlorcyclohexancarboxylat
4'-Ethoxybiphenyl-4-yl-1-cyan-3-chlorcyclohexancarboxylat
4'-Propoxybiphenyl-4-yl-1-cyan-3-chlorcyclohexancarboxylat

4'-Butoxybiphenyl-4-yl-1-cyan-3-chlorcyclohexancarboxylat
4'-Pentoxybiphenyl-4-yl-1-cyan-3-chlorcyclohexancarboxylat
4'-Hexoxybiphenyl-4-yl-1-cyan-3-chlorcyclohexancarboxylat
4'-Heptoxybiphenyl-4-yl-1-cyan-3-chlorcyclohexancarboxylat.

Beispiel 2

Zu einem Gemisch aus 3,1 g 3-Methylcyclohexancarbonitril und 30 ml THF gibt man bei −78° eine Lösung von Lithiumdiisopropylamid (hergestellt aus 3,3 g Diisopropylamin, 30 ml THF und 19 ml 1,6 molarer Butyllithium-Lösung in Hexan) und dann eine Lösung von 9,7 g 1-Brom-2-(4'-pentylbiphenyl-4-yl)ethan in 30 ml THF. Die Aufarbeitung erfolgt analog Beispiel 1 und man erhält 1-(1-Cyan-3-methylcyclohexyl)-2-(4'-pentylbiphenyl-4-yl)ethan, Fp. 73°, $[\alpha]_D^{20} = +1,1°$.

Analog werden hergestellt:

1-(1-Cyan-3-methylcyclohexyl)-2-(4'-ethylbiphenyl-4-yl)-ethan
1-(1-Cyan-3-methylcyclohexyl)-2-(4'-propylbiphenyl-4-yl)-ethan
1-(1-Cyan-3-methylcyclohexyl)-2-(4'-butylbiphenyl-4-yl)-ethan
1-(1-Cyan-3-methylcyclohexyl)-2-(4'-hexylbiphenyl-4-yl)-ethan
1-(1-Cyan-3-methylcyclohexyl)-2-(4'-heptylbiphenyl-4-yl)-ethan
1-(1-Cyan-3-methylcyclohexyl)-2-(4'-ethoxybiphenyl-4-yl)-ethan
1-(1-Cyan-3-methylcyclohexyl)-2-(4'-propoxybiphenyl-4-yl)-ethan
1-(1-Cyan-3-methylcyclohexyl)-2-(4'-butoxybiphenyl-4-yl)-ethan
1-(1-Cyan-3-methylcyclohexyl)-2-(4'-pentoxybiphenyl-4-yl)-ethan
1-(1-Cyan-3-methylcyclohexyl)-2-(4'-hexoxybiphenyl-4-yl)-ethan
1-(1-Cyan-3-methylcyclohexyl)-2-(4'-heptoxybiphenyl-4-yl)-ethan
1-(1-Cyan-3-ethylcyclohexyl)-2-(4'-ethylbiphenyl-4-yl)-ethan
1-(1-Cyan-3-ethylcyclohexyl)-2-(4'-propylbiphenyl-4-yl)-ethan
1-(1-Cyan-3-ethylcyclohexyl)-2-(4'-butylbiphenyl-4-yl)-ethan
1-(1-Cyan-3-ethylcyclohexyl)-2-(4'-pentylbiphenyl-4-yl)-ethan
1-(1-Cyan-3-ethylcyclohexyl)-2-(4'-hexylbiphenyl-4-yl)-ethan
1-(1-Cyan-3-ethylcyclohexyl)-2-(4'-heptylbiphenyl-4-yl)-ethan
1-(1-Cyan-3-ethylcyclohexyl)-2-(4'-ethoxybiphenyl-4-yl)-ethan
1-(1-Cyan-3-ethylcyclohexyl)-2-(4'-propoxybiphenyl-4-yl)-ethan
1-(1-Cyan-3-ethylcyclohexyl)-2-(4'-butoxybiphenyl-4-yl)-ethan
1-(1-Cyan-3-ethylcyclohexyl)-2-(4'-pentoxybiphenyl-4-yl)-ethan
1-(1-Cyan-3-ethylcyclohexyl)-2-(4'-hexoxybiphenyl-4-yl)-ethan
1-(1-Cyan-3-ethylcyclohexyl)-2-(4'-heptoxybiphenyl-4-yl)-ethan.

Beispiel 3

Analog Beispiel 2 erhält man 1-(1-Cyan-3-methylcyclohexyl)-2-[4-(4-pentylcyclohexyl)phenyl-1-yl]-ethan, Fp. 92°, durch Umsetzung von 1-Brom-2-[4-(4-pentylcyclohexyl)phenyl-1-yl]-ethan mit 3-Methylcyclohexancarbonitril.

Analog werden hergestellt:

1-(1-Cyan-3-methylcyclohexyl)-2-[4-(4-ethylcyclohexyl)-phenyl-1-yl]ethan
1-(1-Cyan-3-methylcyclohexyl)-2-[4-(4-propylcyclohexyl)-phenyl-1-yl]ethan
1-(1-Cyan-3-methylcyclohexyl)-2-[4-(4-butylcyclohexyl)-phenyl-1-yl]ethan
1-(1-Cyan-3-methylcyclohexyl)-2-[4-(4-hexylcyclohexyl)-phenyl-1-yl]ethan
1-(1-Cyan-3-methylcyclohexyl)-2-[4-(4-heptylcyclohexyl)-phenyl-1-yl]ethan.

Beispiel A

Eine flüssigkristalline Phase, bestehend aus

24% 4-(5-Heptylpyrimidin-2-yl)-phenyl-(p-pentylbenzyl)ether
24% 4-(5-Heptylpyrimidin-2-yl)-phenyl-(p-heptylbenzyl)ether
18% trans-4-Heptylcyclohexancarbonsäure-(p-octoxyphenylester)
15% trans-4-Heptylcyclohexancarbonsäure-(p-nonoxyphenylester)
10% trans-4-Pentylcyclohexancarbonsäure-(p-hexoxyphenylester)
9% 1-Hydroxy-1-[r-1-cyan-cis-4-(trans-4-pentylcyclohexyl)cyclohexyl]-3-methylcyclohexan

hat K 0° Sc* 45° $S_A$ 54° Ch 61,5° I.

Beispiel B

Eine flüssigkristalline Phase, bestehend aus

3% 2-p-Hexyloxyphenyl-5-heptylpyrimidin,
3% 2-p-Heptyloxyphenyl-5-heptylpyrimidin,
3% 2-p-Octyloxyphenyl-5-heptylpyrimidin,
3% 2-p-Nonyloxyphenyl-5-heptylpyrimidin,
7% 2-p-Hexyloxyphenyl-5-nonylpyrimidin,
27% 2-p-Nonyloxyphenyl-5-nonylpyrimidin,

26% r-1-Cyan-1-butyl-cis-4-(4'-octyloxy-biphenyl-4-yl)cyclohexan,
13% r-1-Cyan-1-hexyl-cis-4-(4'-heptylbiphenyl-4-yl)cyclohexan,
5% r-1-Cyan-cis-1-(4-pentylcyclohexyl)-trans-4-(4-pentylcyclohexyl)cyclohexan
und
10% 4'-Hexyl-biphenyl-4-yl-1-cyan-3-methylcyclohexancarboxylat hat K $-9°$ $S_C^*$ 61° $S_A$ 69° Ch 81° I und eine Spontanpolarisation $P_S = 7$ nC/cm$^2$.

Beispiel C

Eine flüssigkristalline Phase, bestehend aus
10% r-1-Cyan-1-pentyl-cis-4-(4'-butylbiphenyl-4-yl)cyclohexan,
43% r-1-Cyan-1-pentyl-cis-4-(4'-octylbiphenyl-4-yl)cyclohexan,
42% r-1-Cyan-1-butyl-cis-4-(4'-octyloxybiphenyl-4-yl)cyclohexan
und
5% 1-Hydroxy-1-[2-1-cyan-cis-4-(trans-4-pentylcyclohexyl)cyclohexyl]-3-methylcyclohexan
hat K 30° $S_C^*$ 65° $S_A$ 90° Ch 125 I und $P_s = 1$ nC/cm$^2$.

Beispiel D

Eine flüssigkristalline Phase bestehend aus
2% 2-p-Hexyloxyphenyl-5-heptylpyrimidin,
7% 2-p-Hexyloxyphenyl-5-nonylpyrimidin,
2% 2-p-Heptyloxyphenyl-5-heptylpyrimidin,
2% 2-p-Octyloxyphenyl-5-heptylpyrimidin,
25% 2-p-Nonyloxyphenyl-5-nonylpyrimidin,
2% 2-p-Nonyloxyphenyl-5-heptylpyrimidin,
30% r-1-Cyan-1-octyl-cis-4-(4'-octyloxybiphenyl-4-yl)-cyclohexan,
5% r-1-Cyan-1-heptyl-cis-4-(4'-hexylbiphenyl-4-yl)-cyclohexan,
14% optisch aktives r-1-Cyan-1-(2-methylbutyl)-cis-4-(4'-heptyloxybiphenyl-4-yl)-cyclohexan
und
11% optisch aktives 1-(1-Cyan-3-methylcyclohexyl)-2-(4'-pentylbiphenyl-4-yl)-ethan
zeigt K $< -30$ $S_C^*$ 65 $S_A$ 71 Ch 87 I und eine Spontanpolarisation $P_s = +32{,}7$ nC/cm$^2$.

**Patentansprüche**

1. Optisch aktive Verbindungen der Formel I

$$R^1—A^1—Z^1—A^2—R^2 \qquad I$$

worin

$$A^1\ A,\ A^4—A \text{ oder } A—A^4,$$

$R^1$ und $R^2$ jeweils H, eine Alkylgruppe mit 1—12 C-Atomen, worin auch eine oder zwei nicht benachbarte $CH_2$-Gruppen durch O-Atome und/oder —CO-Gruppen und/oder —CO—O-Gruppen und/oder —CH=CH-Gruppen und/oder —CHHalogen- und/oder —CHCN-Gruppen ersetzt sein können, F, Cl, Br, CN oder $R^3—A^3—Z^2$,

A eine in 2-, 3-, 5- oder 6-Stellung durch Hydroxy, Halogen, Nitril und/oder eine Alkylgruppe oder eine fluorierte Alkylgruppe mit jeweils 1—10 C-Atomen, worin auch eine oder zwei nicht benachbarte $CH_2$-Gruppen durch —O—, —CO—, —O—CO—, —CO—O— und/oder —CH=CH— ersetzt sein können, substituierte 1,4-Cyclohexylen- oder 1,4-Cyclohexenylen-Gruppe, worin auch eine oder zwei nicht benachbarte $CH_2$-Gruppen durch —O— und/oder —S— ersetzt sein können, die gegebenenfalls auch in 1- und/oder 4-Stellung durch Hydroxy, Halogen, Nitril und/oder eine Alkylgruppe oder eine fluorierte Alkylgruppe mit jeweils 1—4 C-Atomen, worin auch eine oder zwei nicht benachbarte $CH_2$-Gruppen durch —O—, —CO—, —O—CO—, —CO—O— und/oder —CH=CH— ersetzt sein können, substituiert sein kann,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte $CH_2$-Gruppen durch O-Atome und/oder S-Atome ersetzt sein können, Piperidin-1,4-diyl, 1,4-Bicyclo(2,2,2)-octylen-, Decahydronaphthalin-2,6-diyl- oder 1,2,3,4-Tetrahydronaphthalin-2,6-diyl-Gruppen,

$Z^1$ und $Z^2$ jeweils —CO—O—, —O—CO—, —$CH_2CH_2$—, $OCH_2$—, —$CH_2O$— oder eine Einfachbindung,

$R^3$ H, eine Alkylgruppe mit 1—12, worin auch eine oder zwei nicht benachbarte $CH_2$-Gruppen durch O-Atome und/oder —CO-Gruppen und/oder —CO—O-Gruppen und/oder —CH=CH-Gruppen ersetzt sein können, F, Cl, Br oder CN

bedeutet.

2. Chirale getiltete smektische flüssigkristalline Phase mit mindestens zwei flüssigkristallinen

Komponenten, dadurch gekennzeichnet, daß sie mindestens eine optisch aktive Verbindung der Formel I nach Anspruch 1 enthält.

3. Verwendung der Verbindungen der Formel I nach Anspruch 1 als Komponenten flüssigkristalliner Phasen.

4. Elektrooptisches Anzeigeelement, dadurch gekennzeichnet, daß es als Dielektrikum eine Phase nach Anspruch 2 enthält.

**Revendications**

1. Composés optiquement actifs de formule I:

$$R^1—A^1—Z^1—A^2—R^2 \qquad (I)$$

dans laquelle

$A^1$ A $A^4$—A ou $AA^4$

$R^1$ et $R^2$ représentent chacun H, un groupe alkyle avec 1—12 atomes de carbone, dans lequel aussi un ou deux groupes —$CH_2$— non contigus peuvent être remplacés par des atomes O et/ou des groups —CO et/ou des groupes —CO—O et/ou des groupes —CH=CH— et/ou des groupes —CH halogène- et/ou des groupes —CHCN—, F, Cl, Br, CN ou $R^3$—$A^3$—$Z^2$,

A représente un groupe 1,4-cyclohexylène ou 1,4-cyclohexènylène substitué en position 2-, 3-, 5- ou 6-par un hydroxy, halogène, nitrile et/ou un groupe alkyle ou un groupe alkyle fluoré avec chacun 1—10 atomes de C, dans lequel aussi un ou deux groupes —$CH_2$— non contigus peuvent être remplacés par —O—, —CO— —O—CO—, —CO—O— et/ou —CH=CH—, dans lesquels aussi un ou deux groupes —$CH_2$— non contigus peuvent être remplacés par —O— et/ou —S—, qui le cas échéant aussi peuvent être substitués en position 1 ou 4 par un hydroxy, halogène, nitrile et/ou un groupe alkyle ou un groupe alkyle fluoré avec chacun 1—4 atomes de C, dans lesquels aussi un ou deux groupes —$CH_2$— non contigus peuvent être remplacés par —O—, —CO—, —O—CO—, —CO—O— et/ou —CH=CH—

$A^2$, $A^3$ et $A^4$ représentent chacun un 1,4-phénylène non substitué ou substitué par un ou deux atomes de F et/ou de Cl et/ou des groupes —$CH_3$ et/ou des groupes CN, dans lequel aussi un ou deux groupes —CH— peuvent être remplacés par N, un 1,4- cyclohexylène, dans lequel aussi un ou deux groupes —$CH_2$— non contigus peuvent être remplacés par des atomes de 0 et/ou des atomes de S, des groupes pipéridine-1,4-diyle, 1,4-bicyclo-2,2,2)-octylène, décahydronaphtalène-2,6-diyle ou 1,2,3,4-tétrahydro-naphtalène-2,6-diyle,

$Z^1$ et $Z^2$ représentent chacun —CO—O—, —O—CO—, —$CH_2$—$CH_2$—, —$OCH_2$—, —$CH_2O$— ou une liaison simple,

$R^3$ représente H, un groupe alkyle avec 1—12 atomes de C, dans lequel aussi un ou plusieurs groups —$CH_2$— non contigus peuvent être remplacés par des atomes O et/ou des groupes —CO— et/ou des groupes —CO—O— et/ou des groupes —CH=CH—, F, Cl, Br ou CN.

2. Phase de cristaux liquides smectique chirale avec au moins deux composants de cristaux liquides, caractérisée en ce qu'elle contient au moins un composé optiquement actif de formule I selon la revendication 1.

3. Utilisation de composés de formule I selon la revendication 1 comme composants de phases de cristaux liquides.

4. Elément d'affichage électro-optique caractérisé en ce qu'il contient comme diélectrique une phase selon la revendication 2.

**Claims**

1. Optically active compounds of the formula I:

$$R^1—A^1—Z^1—A^2—R^2 \qquad (I)$$

wherein

—$A^1$— is —A—, —$A^4$—A— or —A—$A^4$—

$R^1$ and $R^2$ are each H, an alkyl groupe with 1—12 C atoms, wherein one or two non-adjacent $CH_2$— groups can also be replaced by O atoms and/or —CO— groups and/or —CO—O— groups and/or —CH=CH— groups and/or —CHhalogen- and/or —CHCN— groups, F, Cl, Br, CN or $R^3$—$A^3$—$Z^2$,

A is a 1,4-cyclohexylene or 1,4-cyclohexenylene group, wherein one or two non-adjacent $CH_2$— groups can also be replaced by —O— and/or —S—, which is substituted in the 2-, 3-, 5- or 6-position by hydroxyl, halogen, nitrile and/or an alkyl group or a fluorinated alkyl group with in each case 1—10 C atoms, wherein one or two non-adjacent $CH_2$— groups can also be replaced by —O—, —CO— —O—CO—, —CO—O— and/or —CH=CH—, and which can optionally also be substituted in the 1- and/or 4-position by hydroxyl, halogen, nitrile and/or an alkyl group or a fluorinated alkyl group with in each case 1—4 C atoms, wherein

one or two non-adjacent $CH_2$— groups can also be replaced by —O—, —CO—, —O—CO—, —CO—O— and/or —CH=CH—,

$A^2$, $A^3$ and $A^4$ are each 1,4-phenylene which is unsubstituted or substituted by one or two F and/or Cl atoms and/or $CH_3$— groups and/or CN— groups, wherein one or two CH— groups can also be replaced by N, 1,4-cyclohexylene, wherein one or two non-adjacent $CH_2$— groups can be replaced by O atoms and/or S atoms, piperidine-1,4-diyl, 1,4-bicyclo(2,2,2)-octylene, decahydronaphthalene-2,6-diyl or 1,2,3,4-tetrahydronaphthalene-2,6-diyl groups,

$Z^1$ and $Z^2$ are each —CO—O—, —O—CO—, —$CH_2CH_2$—, $OCH_2$—, —$CH_2O$— or a single bond and

$R^3$ is H, an alkyl group with 1—12 C atoms, wherein one or two non-adjacent $CH_2$ groups can also be replaced by O atoms and/or —CO— groups and/or —CO—O— groups and/or —CH=CH— groups, F, Cl, Br or CN.

2. Chiral tilted smectic liquid crystal phase with at least two liquid crystal components, characterized in that it contains at least one optically active compound of the formula I according to Claim 1.

3. Use of the compounds of the formula I according to Claim 1 as components of liquid crystal phases.

4. Electrooptical display element, characterized in that it contains a phase according to Claim 2 as the dielectric.